# FASCICULE DE BREVET EUROPEEN

(11) **EP 0 663 909 B1**
(45) Date de publication et mention de la délivrance du brevet: **08.01.1997**
(21) Numéro de dépôt: 93921986.1
(22) Date de dépôt: 04.10.1993
(51) Int. Cl.: C07D 305/14

(54) **PROCEDE D'OBTENTION DE LA DESACETYL-10 BACCATINE III**
VERFAHREN ZUR GEWINNUNG VON 10-DEACETYLBACCATIN-III
METHOD OF OBTAINING 10-DEACETYLBACCATINE III

(30) Priorité: 05.10.1992 FR 9211745
(43) Date de publication de la demande: 26.07.1995
(73) Titulaire: RHONE-POULENC RORER S.A., 92160 Antony (FR)
(72) Inventeur: MARGRAFF, Rodolphe, F-91170 Viry-Châtillon (FR)
(74) Mandataire: Pilard, Jacques
(86) Numéro de dépôt international: FR9300971
(87) Numéro de publication internationale: WO9407881

(56) Documents cités:
- WO-A-94/21622
- JOURNAL OF NATURAL PRODUCTS, vol. 49, no. 4 , Juillet 1986 Columbus, Ohio, US, pages 665 - 669 C.H.O. HUANG, ET AL.: 'New taxanes from Taxus brevifolia, 2' cité dans la demande
- COMPTES RENDUS DE L'ACADEMIE DES SCIENCES. SERIE II: MECANIQUE, PHYSIQUE, CHIMIE, SCIENCES DE L'UNIVERS, SCIENCES DE LA TERRE, vol. 299, no. 15 , 21 Novembre 1984 Montreuil, FR, pages 1039 - 1043 V. SENILH, ET AL.: 'Hémisynthèse de nouveaux analogues du taxol. Etude de leur interacion avec la tubuline'

## Description

La présente invention concerne un procédé pour obtenir des intermédiaires utiles pour la préparation par des procédés semi-synthétiques du taxol, du Taxotère ou de leurs analogues à partir des différentes parties de plantes contenant ces intermédiaires.

Plus particulièrement, l'invention concerne l'obtention sélective de la désacétyl-10 baccatine III à partir de l'écorce, du tronc, des racines ou des feuilles de différentes espèces d'ifs.

Le taxol et le Taxotère ainsi que leurs analogues de formule générale : 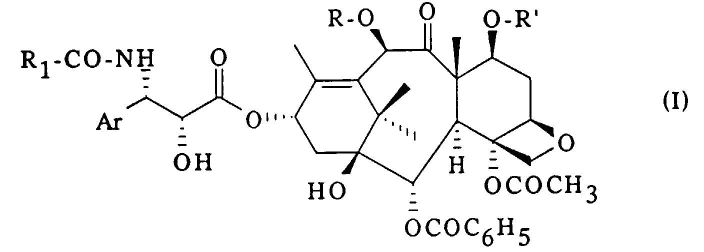 qui manifestent des propriétés anticancéreuses et antileucémiques remarquables, constituent des agents chimiothérapeutiques remarquables pour le traitement d'un certain nombre de cancers tels que par exemple les cancers du sein, de la prostate, du colon, de l'estomac, du rein ou des testicules et plus spécialement le cancer de l'ovaire.

Notamment, dans la formule générale (I), Ar peut représenter un radical phényle éventuellement substitué, R peut représenter un atome d'hydrogène ou un radical acétyle ou un radical carbamoyle N-substitué, R' représente un atome d'hydrogène ou un radical carbamoyle N-substitué et R₁ peut représenter un radical phényle ou un radical R₂-O- dans lequel R₂ représente un radical alcoyle, alcényle, alcynyle, cycloalcoyle, cycloalcényle, bicycloalcoyle, phényle ou hétérocyclyle.

Le taxol correspond au produit de formule générale (I) dans laquelle Ar et R₁ représentent un radical phényle et R représente un radical acétyle et R' représente un atome d'hydrogène et le Taxotère corespond au produit de formule générale (I) dans laquelle Ar représente un radical phényle, R et R' représentent un atome d'hydrogène et R₁ représente un radical t.butoxy.

Le taxol, qui existe à l'état naturel dans différentes espèces d'ifs dans lesquelles il se trouve en faibles quantités, est difficile à isoler sans procéder à la destruction complète de la plante. Par exemple, le taxol peut être isolé selon la méthode de C.H.O. HUANG et coll. J. Natl. Prod., 49, 665 (1986) qui consiste à traiter l'écorce broyée de Taxus brevifolia par le méthanol, à concentrer l'extrait, extraire le concentrat par le dichlorométhane, à concentrer à nouveau, à disperser le résidu dans un mélange hexane-acétone (1-1 en volumes), à purifier la partie soluble par chromatographie sur une colonne de Florisil pour obtenir le taxol brut qui est purifié par recristallisations successives dans des mélanges méthanol-eau et hexane-acétone puis par chromatographie et nouvelle cristallisation. Les quantités de taxol ainsi extraites peuvent représenter de 0,005 à 0,017 % de la partie de la plante mise en oeuvre.

Le Taxotère qui n'existe pas à l'état naturel peut être préparé par hémisynthèse à partir de la désacétyl-10 baccatine III de formule : 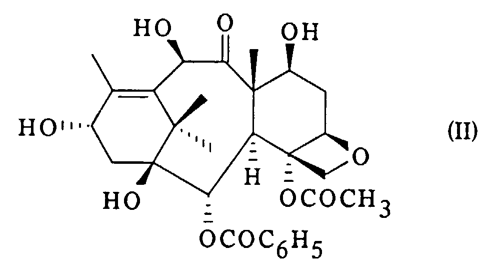 selon les procédés qui sont décrits, par exemple, dans les brevets américains US 4,814,470 ou US 4,924,012 ou dans la demande internationale PCT WO 92/09589.

Le taxol peut aussi être préparé par des procédés qui font intervenir la mise en oeuvre de la désacétyl-10 baccatine III soit par passage par l'intermédiaire du Taxotère dans les conditions décrites dans le brevet américain US 4,857,653 soit par estérification de la baccatine III dans les conditions décrites dans les brevets européens EP 400,971 ou EP 428,376 soit par estérification de la désacétyl-10 baccatine III et acétylation dans les conditions décrites dans les brevet américain US 4,924,011.

Les différentes variétés d'ifs (Taxus baccata, Taxus brevifolia, Taxus canadensis, Taxus cupidata, Taxus floridana, Taxus media, Taxus wallichiana) contiennent des dérivés du taxane dont les principaux sont essentiellement le taxol et la désacétyl-10 baccatine III, les autres dérivés étant plus particulièrement la céphalomannine, la désacétyl-10 céphalomannine ou la baccatine III éventuellement liés à des sucres.

Alors que le taxol se trouve principalement dans le tronc et l'écorce, la désacétyl-10 baccatine III est présente essentiellement dans les feuilles. Par ailleurs, la teneur en désacétyl-10 baccatine III dans les feuilles est généralement très supérieure à celle du taxol, que celui-ci soit présent dans l'écorce, le tronc ou dans les feuilles.

Il en résulte qu'il est particulièrement important de pouvoir disposer de désacétyl-10 baccatine III qui est essentielle à la préparation de quantités de taxol beaucoup plus importantes que par extraction directe à partir des ifs ainsi qu'à la préparation du Taxotère.

En extrayant la désacétyl-10 baccatine III à partir des feuilles d'ifs, on n'est pas conduit à une destruction totale de la plante dont les feuilles peuvent être utilisées à nouveau après chaque cycle de croissance.

Généralement, les méthodes connues d'extraction des dérivés du taxane contenus dans les différentes parties de l'if (écorce, tronc, racines, feuilles ....) nécessitent la mise en oeuvre de techniques chromatographiques iongues et coûteuses qui ne permettent pas une séparation totale et quantitative des dérivés du taxane présents initialement dans la plante.

La désacétyl-10 baccatine III peut être obtenue, avec des rendements voisins de 300 mg par kg de feuilles (Taxus baccata), par un procédé qui met en oeuvre une macération des aiguilles dans l'éthanol, une extraction par un solvant organique tel que le chlorure de méthylène et des chromatographies successives selon le procédé qui est décrit dans le brevet américain US 4,814,470.

Les différents constituants dérivés du taxane présents dans les différentes parties de l'if peuvent aussi être séparés par des méthodes mettant en oeuvre la chromatographie en phase liquide "reverse" qui sont décrites, en particulier dans la demande international PCT WO 92/07842. Ces procédés consistent essentiellement à traiter les extraits bruts d'ifs par chromatographie en phase liquide "reverse" sur un adsorbant sur lequel se fixent les dérivés du taxane, à éluer les dérivés du taxane et à les isoler. Selon ce procédé, il est possible d'isoler 200 mg de désacétyl-10 baccatine III à partir de 1 kg de feuilles broyées et séchées. Un procédé de purification de la désacétyl-10 baccatine III, qui a été extraite des feuilles d'ifs, par chromatographie de partage à contre-courant a été décrit dans la demande WO 94/21622.

Il a maintenant été trouvé, et c'est ce qui fait l'objet de la présente invention, que la désacétyl-10 baccatine III peut être extraite très sélectivement, et avec un excellent rendement, à partir des différentes parties de l'if, et plus particulièrement des feuilles, par un procédé simple ne mettant pas en oeuvre des techniques chromatographiques. Par exemple, il est possible d'extraire environ 800 mg de désacétyl-10 baccatine III par kg de feuilles d'if (Taxus baccata).

Le procédé selon l'invention consiste à isoler la désacétyl-10 baccatine III à partir d'un extrait aqueux de feuilles d'if.

Plus particulièrement le procédé selon l'invention consiste,
- ou bien
   1) à traiter par l'eau les parties broyées de l'if (Taxus sp.),
   2) à séparer la solution aqueuse, contenant la désacétyl-10 baccatine III, de la masse végétale en suspension,
   3) à extraire la désacétyl-10 baccatine III de la solution aqueuse par un solvant organique,
   4) à séparer l'extrait organique, contenant la désacétyl-10 baccatine III, de la phase aqueuse,
   5) à éliminer le solvant organique de l'extrait organique ainsi séparé,
   6) à faire cristalliser sélectivement la désacétyl-10 baccatine III à partir du résidu ainsi obtenu dans un solvant organique,
   7) à isoler la désacétyl-10 baccatine III sous forme purifiée.
- ou bien
   1) à traiter par l'eau les parties broyées de l'if (Taxus sp.),
   2) à séparer la solution aqueuse, contenant la désacétyl-10 baccatine III, de la masse végétale en suspension,
   3) à adsorber la solution aqueuse contenant la désacétyl-10 baccatine III sur un support convenable
   4) à désorber la désacétyl-10 baccatine III au moyen d'un solvant organique 5) à éliminer le solvant organique du désorbat
   6) à faire cristalliser sélectivement la désacétyl-10 baccatine III à partir du résidu ainsi obtenu dans un solvant organique
   7) à isoler la désacétyl-10 baccatine III sous forme purifiée

Le procédé selon l'invention peut être mis en oeuvre sur toute partie appropriée de l'if telle que l'écorce, le tronc, les racines ou les feuilles. L' if utilisé pour la mise en oeuvre du procédé selon l'invention appartient de préférence à la variété Taxus baccata, Taxus brevifolia, Taxus canadensis, Taxus cupidata Taxus floridana, Taxus media ou Taxus wallichiana. Il est particulièrement avantageux d'utiliser les feuilles d'if (Taxus baccata, Taxus brevifolia) qui généralement sont plus riches en désacétyl-10 baccatine III. Les fragments utilisés peuvent avoir des dimensions variant de 0,5 à quelques millimètres. Pour des questions de commodité, il peut être avantageux d'utiliser des fragments dont les dimensions moyennes sont inférieures à 1 mm. Les parties broyées, et éventuellement séchées, de l'if peuvent être obtenues par des opérations de broyage, et éventuellement de séchage, qui, éventuellement, précèdent ou suivent des opérations de congélation et de décongélation des parties fraîches de la plante ou s'intercalent dans des opérations de congélation et de décongélation des parties fraîches de la plante.

Le traitement par l'eau des parties broyées de l'if est effectué selon les techniques connues de l'homme de l'art. En particulier, la solution aqueuse contenant la désacétyl-10 baccatine III est généralement obtenue par agitation, à une température comprise entre 20 et 65°C, des parties broyées de l'if dans de l'eau pendant 30 minutes à 2 heures. La quantité d'eau utilisée peut varier dans de larges limites, cependant il est convenable d'utiliser une quantité d'eau de 2 à 10 litres calculée par kg de partie de plante broyée et séchée et de préférence environ 5 litres d'eau par kg de partie de plante à traiter. Il peut être avantageux d'utiliser de l'eau déminéralisée pour effectuer ce traitement et d'opérer sous ultra-sons.

Dans le but d'améliorer le rendement, il peut être avantageux d'effectuer plusieurs traitements à l'eau de la masse végétale afin d'obtenir des solutions aqueuses dont on extrait la désacétyl-10 baccatine III dans les conditions décrites ci-après.

La solution aqueuse obtenue contenant la désacétyl-10 baccatine III est séparée de la masse végétale par les techniques habituelles telles que la filtration, la centrifugation ou la décantation. La solution aqueuse résultante, éventuellement refroidie, peut être traitée selon l'une des manières suivantes :
1) la désacétyl-10 baccatine III est extraite, en une ou plusieurs fois, par un solvant organique. Les solvants organiques qui conviennent particulièrement bien sont choisis parmi les éthers tels que le méthyl t.butyléther, l'éthyl t.butyléther, le méthyl n.butyléther, le méthyl n.amyléther, l'éthyl t.amyléther, le t.butyl isopropyléther, l'éthyl isobutyléther, le t.butyl n.propyléther ou l'éthyl n.hexyléther et les esters aliphatiques tels que l'acétate d'éthyle, l'acétate de propyle, l'acétate d'isopropyle, l'acétate de n.butyle, l'acétate de t.butyle, le t.butylacétate de méthyle, le propionate de t.butyle ou l'acétate de t.amyle. D'un intérêt tout particulier sont le méthyl t.butyléther, l'éthyl t.butyléther, l'acétate d'éthyle ou l'acétate de n.butyle.
   L'extraction par un solvant organique est généralement effectuée sur une solution aqueuse dont le pH est inférieur à 7 et de préférence inférieur à 6.
   Les extraits organiques, contenant la désacétyl-10 baccatine III, sont séparés de la phase aqueuse par application des techniques habituelles telles que la décantation.
   Les extraits organiques sont éventuellement lavés au moyen d'une solution aqueuse d'une base faible (solution aqueuse de carbonate de sodium par exemple) et/ou à l'eau. Après séchage, le solvant organique de l'extrait est éliminé selon les méthodes habituelles et en particulier par distillation éventuellement sous pression réduite, pour donner un résidu généralement solide dont on isole la désacétyl-10 baccatine III.
2) la solution aqueuse est adsorbée sur un support convenable afin de fixer la désacétyl-10 baccatine III. Comme support, on utilise un résine adsorbante qui est choisie de préférence parmi les résines polystyrène-divinylbenzène.

La désorption de la désacétyl-10 baccatine III est effectuée par lavage du support par un solvant approprié. Comme solvant, on utilise de préférence un alcool aliphatique contenant 1 à 3 atomes de carbone et plus particulièrement le méthanol.

La solution organique est séparée du support, généralement par filtration, puis est concentrée à sec généralement par distillation éventuellement sous pression réduite pour donner un résidu généralement solide dont on isole la désacétyl-10 baccatine III.

La cristallisation sélective de la désacétyl-10 baccatine III brute, obtenue selon l'une ou l'autre voie, est effectuée à partir d'une solution du résidu obtenu dans un solvant organique ou dans un mélange de solvants organiques. Comme solvants permettant la cristallisation sélective de la désacétyl-10 baccatine III peuvent être avantageusement utilisés les nitriles tels que l'acétonitrile, le propionitrile ou l'isobutyronitrile éventuellement en mélange avec un alcool aliphatique tel que le méthanol, l'éthanol, le propanol, l'isopropanol ou le n.butanol ou un ester aliphatique tel que l'acétate d'éthyle, l'acétate d'isopropyle, l'acétate de n.butyle ou l'acétate de t.butyle ou une cétone aliphatique telle que l'acétone, la méthyl éthylcétone, la méthyl propylcétone, la méthyl n.butylcétone ou la méthyl isobutylcétone. Il est particulièrement avantageux d'effectuer la cristallisation sélective dans l'acétonitrile éventuellement en présence d'éthanol et/ou d'acétate d'éthyle ou de n.butyle et/ou d'acétone.

La désacétyl-10 baccatine III qui précipite peut être séparée par filtration, décantation ou centrifugation.

Le procédé selon l'invention permet d'obtenir la désacétyl-10 baccatine III pratiquement pure avec des rendements généralement très supérieurs à ceux obtenus par la mise en oeuvre des procédés antérieurement connus. Le procédé selon l'invention permet d'extraire de façon quasiment quantitative la totalité de la désacétyl-10 baccatine III contenue dans les parties de la plante utilisées et en particulier des feuilles.

La désacétyl-10 baccatine III obtenue selon le procédé d'extraction de la présente invention peut être utilisée pour préparer le taxol ou le Taxotère ou leurs dérivés dans les conditions qui sont décrites plus particulièrement dans les brevets EP 0 253 738, EP 0 253 739, EP 0 336 841, EP 0 336 840, WO 92 09589, EP 0 400 971 et EP 0 428 376.

Les exemples suivants illustrent le procédé selon l'invention.

### EXEMPLE 1

A 2,5 litres d'eau déminéralisée chauffés à 50°C, on ajoute 500 g de feuilles d'ifs (Taxus baccata) broyées et séchées dont la taille moyenne des particules est inférieure à 1 mm et dont le titre en désacétyl-10 baccatine III, déterminé par chromatographie liquide à haute performance (HPLC), est de 0,08 % (soit 400 mg de désacétyl-10 baccatine III dans 500 g de feuilles). On agite pendant 1 heure à 50°C puis on filtre.

Le filtrat (1,8 litre), dont le pH est de 5,4, est extrait par 3 fois 0,9 litre d'acétate d'éthyle. Les phases organiques réunies (2,7 litres) sont lavées 2 fois par 1 litre d'une solution de carbonate de sodium 0,1M puis par 2 fois 1 litre d'eau déminéralisée et enfin séchées sur sulfate de sodium. Après filtration et concentration à sec, on obtient un solide (3,2 g) que l'on reprend par 9 cm3 d'acétonitrile à une température voisine de 70°C. Après refroidissement pendant une nuit à une température voisine de +4°C, le précipité est séparé par filtration. On obtient ainsi, après séchage, 245 mg de cristaux contenant, d'après l'analyse par HPLC, 75 % de désacétyl-10 baccatine III pure, soit 183 mg. Les eaux-mères, d'après l'analyse par HPLC, contiennent 40 mg de désacétyl-10 baccatine III.

La masse végétale, qui a retenu 0,7 litre d'eau, est extraite 2 fois dans les conditions décrites ci-dessus. Les filtrats aqueux réunis sont traités comme précédemment. On obtient ainsi 2,5 g d'un produit solide que l'on reprend dans 5 cm3 d'acétonitrile à une température voisine de 70°C. Après refroidissement pendant une nuit à une température voisine de +4°C, on sépare par filtration 169 mg de cristaux contenant, d'après l'analyse par HPLC, 75 % de désacétyl-10 baccatine III pure soit 127 mg. Les eaux-mères contiennent, d'après l'analyse par HPLC, 47 mg de désacétyl-10 baccatine III.

La quantité totale de désacétyl-10 baccatine III extraite par l'eau est de 183 + 40 + 127+ 47 = 397 mg.

Le rendement est pratiquement quantitatif.

### EXEMPLE 2

On chauffe 3 litres d'eau déminéralisée à 50°C puis on ajoute 500 g de feuilles d'ifs broyées et séchées et dont le diamètre moyen des particules est de 1 mm et dont le titre en désacétyl-10 baccatine III, déterminé par chromatographie liquide à haute performance (HPLC), est de 0,08 % (soit 400 mg de désacétyl-10 baccatine III dans 500 g de feuilles). On agite pendant 1 heure à 50°C puis on filtre. Le filtrat (1,97 litre), dont le pH est de 5,15, est ajusté à pH = 4,6 par addition d'acide chlorhydrique concentré puis il est filtré sur une membrane de carton amianté dans un filtre Seitz. On recueille 1,8 litre de filtrat.

On extrait 300 cm3 de filtrat par 2 fois 150 cm3 puis 4 fois 60 cm3 de méthyl tert.butyl éther (MTBE). Les extraits réunis et concentrés fournissent 620 mg de solides qui sont repris par 3 cm3 d'acétonitrile à 70°C.

Après refroidissement pendant une nuit à +4°C, on sépare par filtration 28 mg de désacétyl-10 baccatine III cristallisé dont le titre déterminé par HPLC est de 90 %. Les eaux-mères contiennent 12,8 mg de désacétyl-10 baccatine III (dosage par HPLC).

### EXEMPLE 3

On broye 1 kg de feuilles d'if (Taxus baccata) fraîchement coupées dont la teneur en eau est de 69% et dont la teneur en désacétyl-10 baccatine III est de 651 mg/kg (détermination par HPLC) dans un mixer de 2 litres en présence d'eau déminéralisée à raison de 10 opérations successives identiques en mettant en oeuvre chaque fois 100 g de feuilles et 500 cm3 d'eau à une température voisine de 20°C. Le mélange obtenu est filtré sur toile de coton puis sur papier en rinçant avec au total 200 cm3 d'eau. On recueille 5.000 cm3 de filtrat soit 84,9 % du volume total d'eau (eau mise en oeuvre et eau contenue dans les feuilles). D'après le dosage par HPLC, la solution aqueuse ainsi obtenue contient 72 g de matières sèches dont 550 mg de désacétyl-10 baccatine III soit pratiquement la quantité théorique de 552,7 mg (84,9 % de 651 mg). Le reste de la désacétyl-10 baccatine III contenue dans l'eau imprégnant la matière végétale peut être résupéré par lavage à l'eau de la matière végétale.

La solution aqueuse (5.000 cm3) est percolée sur une colonne de 5 cm de diamètre remplie sur une hauteur de 10 cm avec une résine polystyrène-divinylbenzène (Amberchrom CG 161 md® (Tosohaas, Stuttgart, Allemagne).

La résine est rincée à l'eau, séchée puis mise ensuspension dans du méthanol.

Après filtration et concentration à sec du filtrat, on obtient un résidu (15,5 g) que l'on reprend par 100 cm3 d'acétate d'éthyle. On sépare par filtration 8,7 g d'un produit insoluble puison concentre le filtrat jusqu'à un volume de 5 cm3. On ajoute 30 cm3 d'acétonitrile. On obtient une solutution limpide dans laquelle, très rapidement, apparaissent des cristaux blancs. Après une nuit de repos à 4°C, les cristaux blancs sont séparés par filtration et séchés.On obtient ainsi 548 mg de désacétyl-10 baccatine III solvatée par une mole d'acétonitrile avec un rendement de 92 %.

Un dosage par HPLC avec normalisation interne montre que ces cristaux contiennent 82 % de désacétyl-10 baccatine III.

Le rendement en désacétyl-10 baccatine III est donc de 75,4 %.

## Revendications

1. Procédé de préparation de la désacétyl-10 baccatine III caractérisé en ce que:
1) on traite par l'eau les parties broyées de l'if,
2) on sépare la solution aqueuse, contenant la désacétyl-10 baccatine III, de la masse végétale en suspension,
3) on extrait la désacétyl-10 baccatine III de la solution aqueuse par un solvant organique,
4) on sépare l'extrait organique contenant la désacétyl-10 baccatine III de la phase aqueuse,
5) on élimine le solvant de l'extrait organique ainsi séparé,
6) on fait cristalliser sélectivement la désacétyl-10 baccatine III à partir du résidu ainsi obtenu dans un solvant organique, puis
7) on isole la désacétyl-10 baccatine III sous forme purifiée.

2. Procédé de préparation de la désacétyl-10 baccatine III caractérisé en ce que:
1) on traite par l'eau les parties broyées de l'if (Taxus sp.),
2) on sépare la solution aqueuse, contenant la désacétyl-10 baccatine III, de la masse végétale en suspension,
3) on adsorbe la solution aqueuse contenant la désacétyl-10 baccatine III sur un support convenable
4) on désorbe la désacétyl-10 baccatine III au moyen d'un solvant organique
5) on élimine le solvant organique du désorbat
6) on fait cristalliser sélectivement la désacétyl-10 baccatine III à partir du résidu ainsi obtenu dans un solvant organique
7) on isole la désacétyl-10 baccatine III sous forme purifiée

3. Procédé selon l'une des revendications 1 ou 2 caractérisé en ce que les parties broyées, et éventuellement séchées, de l'if sont agitées avec de l'eau à une température comprise entre 20 et 65°C.

4. Procédé selon la revendication 3 caractérisé en ce que les parties broyées, et éventuellement séchées, de l'if sont obtenues par des opérations de broyage, et éventuellement de séchage, qui, éventuellement, précèdent ou suivent des opérations de congélation et de décongélation des parties fraîches de la plante ou s'intercalent dans des opérations de congélation et de décongélation des parties fraîches de la plante.

5. Procédé selon la revendication 3 caractérisé en ce que l'on utilise de 2 à 10 litres d'eau par kg de partie de plante broyée et séchée.

6. Procédé selon l'une des revendications 3 à 5 caractérisé en ce que l'on utilise de l'eau déminéralisée.

7. Procédé selon l'une des revendications 3 à 5 caractérisé en ce que l'on opére sous ultra-sons.

8. Procédé selon l'une des revendications 1 ou 2 caractérisé en ce que l'on sépare la solution aqueuse contenant la désacétyl-10 baccatine III de la masse végétale en suspension par filtration, centrifugation ou par décantation.

9. Procédé selon la revendication 1 caractérisé en ce que l'on extrait la solution aqueuse contenant la désacétyl-10 baccatine III par un solvant organique choisi parmi les éthers et les esters aliphatiques.

10. Procédé selon la revendication 9 caractérisé en ce que le solvant organique est choisi parmi le méthyl t.butyléther, l'éthyl t.butyléther, le méthyl n.butyléther, le méthyl n.amyléther, l'éthyl t.amyléther, le t.butyl isopropyléther, l'éthyl isobutyléther, le t.butyl n.propyléther, l'éthyl n.hexyléther, l'acétate d'éthyle, l'acétate de propyle, l'acétate d'isopropyle, l'acétate de n.butyle, l'acétate de t.butyle, le t.butylacétate de méthyle, le propionate de t.butyle et l'acétate de t.amyle.

11. Procédé selon la revendication 9 caractérisé en ce que le solvant est choisi parmi méthyl t.butyléther, l'éthyl t.butyléther, l'acétate d'éthyle et l'acétate de n.butyle.

12. Procédé selon la revendication 9 caractérisé en ce que l'on opère avec une solution aqueuse dont le pH est inférieur à 7.

13. Procédé selon la revendication 9 caractérisé en ce que le pH de la solution aqueuse est inférieur à 6.

14. Procédé selon la revendication 1 caractérisé en ce que l'on sépare l'extrait organique contenant la désacétyl-10 baccatine III de la solution aqueuse par décantation.

15. Procédé selon la revendication 1 caractérisé en ce que l'on élimine le solvant de l'extrait organique contenant la désacétyl-10 baccatine III, éventuellement lavé au moyen d'une solution aqueuse d'une base faible et/ou à l'eau, par distillation.

16. Procédé selon la revendication 15 caractérisé en ce que l'élimination du solvant de l'extrait organique est effectuée par distillation sous pression réduite.

17. Procédé selon la revendication 2 caractérisé en ce que l'on adsorbe la solution aqueuse par percolation sur une résine.

18. Procédé selon la revendication 17 caractérisé en ce que la résine est une résine polystyrène-divinylbenzène.

19. Procédé selon la revendication 2 caractérisé en ce que la désorption de la désacétyl-10 baccatine III de la résine est effectuée au moyen d'un solvant organique.

20. Procédé selon la revendication 19 caractérisé en ce que le solvant organique est choisi parmi les alcools aliphatiques contenant 1 à 3 atomes de carbone.

21. Procédé selon la revendication 20 caractérisé en ce que le solvant organique est le méthanol.

22. Procédé selon la revendication 2 caractérisé en ce que la solution organique contenant la désacétyl-10 baccatine III est concentrée à sec.

23. Procédé selon la revendication 22 caractérisé en ce que la concentration est effectuée par distillation éventuellement sous pression réduite.

24. Procédé selon l'une des revendications 1 ou 2 caractérisé en ce que l'on cristallise sélectivement la désacétyl-10 baccatine III, à partir du résidu obtenu après l'élimination du solvant, dans un solvant organique ou dans un mélange de solvants organiques.

25. Procédé selon la revendication 24 caractérisé en ce que le solvant est choisi parmi les nitriles aliphatiques éventuellement en mélange avec un alcool aliphatique ou un ester aliphatique ou une cétone aliphatique.

26. Procédé selon la revendication 25 caractérisé en ce que les nitriles sont choisis parmi l'acétonitrile et le propionitrile.

27. Procédé selon la revendication 25 cractérisé en ce que l'alcool aliphatique est choisi parmi le méthanol, l'éthanol, le propanol, l'isopropanol et le n.butanol.

28. Procédé selon la revendication 25 caractérisé en ce que l'ester aliphatique est choisi parmi l'acétate d'éthyle, l'acétate d'isopropyle, l'acétate de n.butyle et l'acétate de t.butyle.

29. Procédé selon la revendication 25 caractérisé en ce que la cétone aliphatique est choisie parmi l'acétone, la méthyl ethylcétone, la méthyl propylcétone, la méthyl n.butylcétone et la méthyl isobutylcétone.

30. Procédé selon la revendication 25 caractérisé en ce que la cristallisation sélective est effectuée dans l'acétonitrile éventuellement associé à l'éthanol et/ou l'acétate d'éthyle ou de n.butyle et/ou l'acétone.

31. Procédé selon l'une des revendications 1 ou 2 caractérisé en ce que la désacétyl-10 baccatine III est isolée par filtration, décantation ou centrifugation.

32. Procédé selon l'une des revendications 1 à 31 caractérisé en ce que l'on extrait la désacétyl-10 baccatine III à partir de l'écorce, du tronc, des racines ou des feuilles de l'if.

33. Procédé selon l'une des revendications 1 à 31 caractérisé en ce que l'on extrait la désacétyl-10 baccatine III à partir des feuilles de l'if.

34. Procédé selon l'une des revendications 32 ou 33 caractérisé en ce que l'if appartient à la variété Taxus baccata, Taxus brevifolia, Taxus canadensis, Taxus cuspidata, Taxus floridana, Taxus media ou Taxus wallichiana.

## Patentansprüche

1. Verfahren zur Herstellung von 10-Desacetylbaccatin III, dadurch gekennzeichnet, daß man
1) mit Wasser die zerkleinerten Teile der Eibe behandelt,
2) die wäßrige Lösung, die 10-Desacetyl-baccatin III enthält, von der pflanzlichen Masse in Suspension abtrennt,
3) das 10-Desacetyl-baccatin III der wäßrigen Lösung mit einem organischen Lösungsmittel extrahiert,
4) den das 10-Desacetyl-baccatin III der wäßrigen Phase enthaltenden, organischen Extrakt abtrennt,
5) das Lösungsmittel des so abgetrennten, organischen Extrakts eliminiert,
6) selektiv 10-Desacetyl-baccatin III aus dem so erhaltenen Rückstand in einem organischen Lösungsmittel zur Kristallisation bringt und hiernach
7) das 10-Desacetyl-baccatin III in gereinigter Form isoliert.

2. Verfahren zur Herstellung von 10-Desacetyl-baccatin III, dadurch gekennzeichnet, daß man
1) mit Wasser die zerkleinerten Teile der Eibe (Taxus sp.) behandelt,
2) die wäßrige Lösung, die das 10-Desacetyl-baccatin III enthält, von der pflanzlichen Masse in Suspension abtrennt,
3) die das 10-Desacetyl-baccatin III enthaltende, wäßrige Lösung an einem geeigneten Träger adsorbiert,
4) das 10-Desacetyl-baccatin III mit Hilfe eines organischen Lösungsmittels desorbiert,
5) das organische Lösungsmittel des Desorbats eliminiert,
6) selektiv das 10-Desacetyl-baccatin III aus dem so erhaltenen Rückstand in einem organischen Lösungsmittel zur Kristallisation bringt,
7) das 10-Desacetyl-baccatin III im gereinigter Form isoliert.

3. Verfahren gemäß einem der Ansprüche 1 oder 2, dadurch gekennzeichnet, daß die zerkleinerten und gegebenenfalls getrockneten Teile der Eibe mit Wasser bei einer Temperatur zwischen 20 und 65°C gerührt werden.

4. Verfahren gemäß Anspruch 3, dadurch gekennzeichnet, daß die zerkleinerten und gegebenenfalls getrockneten Teile der Eibe durch Maßnahmen der Zerkleinerung und gegebenenfalls Trocknung erhalten werden, die gegebenenfalls Maßnahmen eines Gefrierens und Auftauens der frischen Pflanzenteile vorangehen oder auf diese folgen oder eingeschaltet sind zwischen Maßnahmen eines Gefrierens und Auftauens der frischen Pflanzenteile.

5. Verfahren gemäß Anspruch 3, dadurch gekennzeichnet, daß 2 bis 10 l Wasser/kg zerkleinerte und getrocknete Pflanzenteile verwendet werden.

6. Verfahren gemäß einem der Ansprüche 3 bis 5, dadurch gekennzeichnet, daß man entmineralisiertes Wasser verwendet.

7. Verfahren gemäß einem der Ansprüche 3 bis 5, dadurch gekennzeichnet, daß man unter Ultraschall arbeitet.

8. Verfahren gemäß einem der Ansprüche 1 oder 2, dadurch gekennzeichnet, daß man die das 10-Desacetyl-baccatin III der pflanzlichen Masse in Suspension enthaltende, wäßrige Lösung durch Filtrieren, Zentrifugieren oder Dekantation abtrennt.

9. Verfahren gemäß Anspruch 1, dadurch gekennzeichnet, daß man die das 10-Desacetyl-baccatin III enthaltende, wäßrige Lösung mit einem organischen Lösungsmittel, ausgewählt unter den Ethern und den aliphatischen Estern, extrahiert.

10. Verfahren gemäß Anspruch 9, dadurch gekennzeichnet, daß das organische Lösungsmittel unter Methyl, tert.-Butylether, Ethyl-tert.-butylether, Methyl-n-butylether, Methyl-n-amylether, Ethyl-tert.-amylether, tert.-Butylisopropylether, Ethyl-isobutylether, tert.-Butyl-n-propylether, Ethyl-n-hexylether, Ethylacetat, Propylacetat, Isopropylacetat, n-Butylacetat, tert.-Butylacetat, Methyl-tert.-butylacetat, tert.-Butylpropionat und tert.-Amylacetat ausgewählt wird.

11. Verfahren gemäß Anspruch 9, dadurch gekennzeichnet, daß das Lösungsmittel unter Methyl-tert.-butylether, Ethyl-tert.-butylether, Ethylacetat und n-Butylacetat ausgewählt wird.

12. Verfahren gemäß Anspruch 9, dadurch gekennzeichnet, daß man mit einer wäßrigen Lösung arbeitet, deren pH niedriger als 7 ist.

13. Verfahren gemäß Anspruch 9, dadurch gekennzeichnet, daß der pH der wäßrigen Lösung niedriger als 6 ist.

14. Verfahren gemäß Anspruch 1, dadurch gekennzeichnet, daß man den das 10-Desacetyl-baccatin III enthaltenden, organischen Extrakt von der wäßrigen Lösung durch Dekantation abtrennt.

15. Verfahren gemäß Anspruch 1, dadurch gekennzeichnet, daß man das Lösungsmittel des das 10-Desacetyl-baccatin III enthaltenden, organischen Extrakts, gegebenenfalls gewaschen mit einer wäßrigen Lösung einer schwachen Base und/oder mit Wasser, durch Destillation eliminiert.

16. Verfahren gemäß Anspruch 15, dadurch gekennzeichnet, daß die Eliminierung des Lösungsmittels des organischen Extrakts durch Destillation unter vermindertem Druck durchgeführt wird.

17. Verfahren gemäß Anspruch 2, dadurch gekennzeichnet, daß man die wäßrige Lösung durch Perkolation an einem Harz adsorbiert.

18. Verfahren gemäß Anspruch 17,dadurch gekennzeichnet, daß das Harz ein Polystyrol-Divinylbenzol-Harz ist.

19. Verfahren gemäß Anspruch 2, dadurch gekennzeichnet, daß die Desorption von 10-Desacetyl-baccatin III von dem Harz mit Hilfe eines organischen Lösungsmittels durchgeführt wird.

20. Verfahren gemäß Anspruch 19,dadurch gekennzeichnet, daß das organische Lösungsmittel unter den aliphatischen Alkoholen mit 1 bis 3 Kohlenstoffatomen ausgewählt wird.

21. Verfahren gemäß Anspruch 20,dadurch gekennzeichnet, daß das organische Lösungsmittel Methanol ist.

22. Verfahren gemäß Anspruch 2, dadurch gekennzeichnet, daß die das 10-Desacetyl-baccatin III enthaltende, organische Lösung zur Trockene eingeengt wird.

23. Verfahren gemäß Anspruch 22,dadurch gekennzeichnet, daß das Einengen durch Destillation, gegebenenfalls unter vermindertem Druck,durchgeführt wird.

24. Verfahren gemäß einem der Ansprüche oder 2, dadurch gekennzeichnet, daß man selektiv 10-Desacetyl-baccatin III, ausgehend von dem erhaltenen Harz nach Entfernung des Lösungsmittels, in einem organischen Lösungsmittel oder in einem Gemisch organischer Lösungsmittel kristallisiert.

25. Verfahren gemäß Anspruch 24,dadurch gekennzeichnet, daß das Lösungsmittel unter aliphatischen Nitrilen, gegebenenfalls im Gemisch mit einem aliphatischen Alkohol oder einem aliphatischen Ester oder einem aliphatischen Keton,ausgewählt wird.

26. Verfahren gemäß Anspruch 25,dadurch gekennzeichnet, daß die Nitrile unter Acetonitril und Propionitril ausgewählt werden.

27. Verfahren gemäß Anspruch 25,dadurch gekennzeichnet, daß der aliphatische Alkohol unter Methanol, Ethanol, Propanol, Isopropanol und n-Butanol ausgewählt wird.

28. Verfahren gemäß Anspruch 25,dadurch gekennzeichnet, daß der aliphatische Ester unter Ethylacetat, Isopropylacetat, n-Butylacetat und tert.-Butylacetat ausgewählt wird.

29. Verfahren gemäß Anspruch 25,dadurch gekennzeichnet, daß das aliphatische Keton unter Aceton, Methylethylketon, Methylpropylketon, Methyl-n-butylketon und Methylisobutylketon ausgewählt wird.

30. Verfahren gemäß Anspruch 25,dadurch gekennzeichnet, daß die selektive Kristallisation in Acetonitril, gegebenenfalls gemeinsam mit Ethanol und/oder Ethylacetat oder n-Butylacetat und/oder Aceton durchgeführt wird.

31. Verfahren gemäß einem der Ansprüche 1 oder 2, dadurch gekennzeichnet, daß das 10-Desacetyl-baccatin III durch Filtrieren, Dekantieren oder Zentrifugieren isoliert wird.

32. Verfahren gemäß einem der Ansprüche 1 bis 31, dadurch gekennzeichnet, daß man das 10-Desacetyl-baccatin III aus der Rinde, dem Stamm, den Wurzeln oder den Nadeln der Eibe extrahiert.

33. Verfahren gemäß einem der Ansprüche 1 bis 31, dadurch gekennzeichnet, daß man das 10-Desacetyl-baccatin III aus den Nadeln der Eibe extrahiert.

34. Verfahren gemäß einem der Ansprüche 32 oder 33, dadurch gekennzeichnet, daß die Eibe der Gattung Taxus baccata, Taxus brevifolia, Taxus canadensis, Taxus cuspidata, Taxus floridana, Taxus media oder Taxus wallichiana angehört.

## Claims

1. Process for preparing 10-deacetylbaccatin III, characterized in that:
1) the ground parts of the yew are treated with water,
2) the aqueous solution, containing 10-deacetylbaccatin III, is separated from the suspended plant mass,
3) 10-deacetylbaccatin III is extracted from the aqueous solution with an organic solvent,
4) the organic extract containing 10-deacetylbaccatin III is separated from the aqueous phase,
5) the solvent is removed from the organic extract thus separated,
6) 10-deacetylbaccatin III is selectively crystallized, in an organic solvent, from the residue thus obtained, and then
7) 10-deacetylbaccatin III is isolated in the purified form.

2. Process for preparing 10-deacetylbaccatin III, characterized in that:
1) the ground parts of the yew (Taxus sp.) are treated with water,
2) the aqueous solution, containing 10-deacetylbaccatin III, is separated from the suspended plant mass,
3) the aqueous solution containing 10-deacetyl-baccatin III is adsorbed on a suitable substrate
4) 10-deacetylbaccatin III is desorbed using an organic solvent
5) the organic solvent is removed from the desorbate
6) 10-deacetylbaccatin III is selectively crystallized, in an organic solvent, from the residue thus obtained
7) 10-deacetylbaccatin III is isolated in the purified form.

3. Process according to either of claims 1 and 2, characterized in that the ground, and optionally dried, parts of the yew are stirred with water at a temperature between 20 and 65°C.

4. Process according to claim 3, characterized in that the ground, and optionally dried, parts of the yew are obtained by grinding, and optionally drying, operations which, optionally, precede or follow freezing and defrosting operations of the fresh parts of the plant or are inserted into freezing and defrosting operations of the fresh parts of the plant.

5. Process according to claim 3, characterized in that from 2 to 10 litres of water per kg of ground and dried plant part are used.

6. Process according to one of claims 3 to 5, characterized in that demineralized water is used.

7. Process according to one of claims 3 to 5, characterized in that the process is carried out under ultrasound.

8. Process according to either of claims 1 and 2, characterized in that the aqueous solution containing 10-deacetylbaccatin III is separated from the suspended plant mass by filtration, centrifuging or by settling.

9. Process according to claim 1, characterized in that the aqueous solution containing 10-deacetylbaccatin III is extracted with an organic solvent chosen from ethers and aliphatic esters.

10. Process according to claim 9, characterized in that the organic solvent is chosen from methyl t-butyl ether, ethyl t-butyl ether, methyl n-butyl ether, methyl n-amyl ether, ethyl t-amyl ether, t-butyl isopropyl ether, ethyl isobutyl ether, t-butyl n-propyl ether, ethyl n-hexyl ether, ethyl acetate, propyl acetate, isopropyl acetate, n-butyl acetate, t-butyl acetate, methyl t-butyl acetate, t-butyl propionate and t-amyl acetate.

11. Process according to claim 9, characterized in that the solvent is chosen from methyl t-butyl ether, ethyl t-butyl ether, ethyl acetate and n-butyl acetate.

12. Process according to claim 9, characterized in that the process is carried out with an aqueous solution whose pH is less than 7.

13. Process according to claim 9, characterized in that the pH of the aqueous solution is less than 6.

14. Process according to claim 1, characterized in that the organic extract containing 10-deacetylbaccatin III is separated from the aqueous solution by settling.

15. Process according to claim 1, characterized in that the solvent is removed from the organic extract containing 10-deacetylbaccatin III, optionally washed using an aqueous solution of a weak base and/or with water, by distillation.

16. Process according to claim 15, characterized in that removal of the solvent from the organic extract is carried out by distillation under reduced pressure.

17. Process according to claim 2, characterized in that the aqueous solution is adsorbed by percolation through a resin.

18. Process according to claim 17, characterized in that the resin is a polystyrene/divinylbenzene resin.

19. Process according to claim 2, characterized in that desorption of 10-deacetylbaccatin III from the resin is carried out using an organic solvent.

20. Process according to claim 19, characterized in that the organic solvent is chosen from aliphatic alcohols containing 1 to 3 carbon atoms.

21. Process according to claim 20, characterized in that the organic solvent is methanol.

22. Process according to claim 2, characterized in that the organic solution containing 10-deacetylbaccatin III is concentrated to dryness.

23. Process according to claim 22, characterized in that concentration is carried out by distillation, optionally under reduced pressure.

24. Process according to either of claims 1 or 2, characterized in that 10-deacetylbaccatin III is selectively crystallized, in an organic solvent or in a mixture of organic solvents, from the residue obtained after removal of the solvent.

25. Process according to claim 24, characterized in that the solvent is chosen from aliphatic nitriles, optionally mixed with an aliphatic alcohol or an aliphatic ester or an aliphatic ketone.

26. Process according to claim 25, characterized in that the nitriles are chosen from acetonitrile and propionitrile.

27. Process according to claim 25, characterized in that the aliphatic alcohol is chosen from methanol, ethanol, propanol, isopropanol and n-butanol.

28. Process according to claim 25, characterized in that the aliphatic ester is chosen from ethyl acetate, isopropyl acetate, n-butyl acetate and t-butyl acetate.

29. Process according to claim 25, characterized in that the aliphatic ketone is chosen from acetone, methyl ethyl ketone, methyl propyl ketone, methyl n-butyl ketone and methyl isobutyl ketone.

30. Process according to claim 25, characterized in that selective crystallization is carried out in acetonitrile, optionally in combination with ethanol and/or ethyl or n-butyl acetate and/or acetone.

31. Process according to either of claims 1 or 2, characterized in that 10-deacetylbaccatin III is isolated by filtration, settling or centrifuging.

32. Process according to one of claims 1 to 31, characterized in that 10-deacetylbaccatin III is extracted from the bark, trunk, roots or leaves of the yew.

33. Process according to one of claims 1 to 31, characterized in that 10-deacetylbaccatin III is extracted from the leaves of the yew.

34. Process according to either of claims 32 or 33, characterized in that the yew belongs to the variety Taxus baccata, Taxus brevifolia, Taxus canadensis, Taxus cupidata, Taxus floridana, Taxus media or Taxus wallichiana.
